(19) Europäisches Patentamt
European Patent Office
Office européen des brevets

(11) EP 0 465 358 B1

(12) **FASCICULE DE BREVET EUROPEEN**

(45) Date de publication et mention de la délivrance du brevet: **07.02.1996 Bulletin 1996/06**

(51) Int. Cl.[6]: **C07D 409/04**, C07D 495/04, A61K 31/445 // (C07D495/04, 333:00, 221:00)

(21) Numéro de dépôt: **91401833.8**

(22) Date de dépôt: **03.07.1991**

(54) **Dérivé thiényl-2 glycidique, son procédé de préparation et son utilisation comme intermédiaire de synthèse**

2-Thienylglycidsäurederivat, Verfahren zu seiner Herstellung und seine Verwendung als Synthesezwischenprodukt

2-Thienylglycidic acid derivative, process for its preparation and its use as synthetic intermediate

(84) Etats contractants désignés:
**AT BE CH DE DK ES FR GB GR IT LI LU NL SE**

(30) Priorité: **04.07.1990 FR 9008482**

(43) Date de publication de la demande:
**08.01.1992 Bulletin 1992/02**

(73) Titulaire: **SANOFI**
**F-75008 Paris (FR)**

(72) Inventeurs:
- **Bousquet, André**
**F-04200 Sisteron (FR)**
- **Calet, Serge**
**F-04200 Sisteron (FR)**
- **Heymes, Alain**
**F-04200 Sisteron (FR)**

(74) Mandataire: **Obolensky, Michel et al**
**F-75441 Paris Cédex 09 (FR)**

(56) Documents cités:
**EP-A- 0 069 002**

- **PATENT ABSTRACTS OF JAPAN, vol. 11, no. 317 (C-452)[2764], 15 octobre 1987; & JP-A-62 103 087 (TOYO PHARMA K.K.) 13-05-1987**
- **CHEMICAL ABSTRACTS, vol. 89, no. 3, 17 juillet 1978, page 650, abrégé no. 24280e, Columbus, Ohio, US;& JP-A-53 07 687 (SANWA CHEMICAL LABORATORIES) 24-01-1978**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.9.9/3.4

## Description

La présente invention se rapporte, d'une manière générale, à un nouveau dérivé thiényl-2 glycidique, à son procédé de préparation ainsi qu'à son utilisation comme intermédiaire de synthèse.

Plus précisément, l'invention a pour objet le thiényl-2 glycidate d'isopropyle de formule :

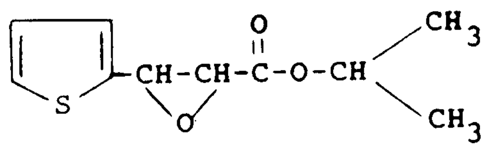

I

Le composé de l'invention s'est révélé particulièrement utile comme produit intermédiaire notamment pour la préparation de la thiényl-2 acétaldoxime. Cette dernière peut être elle-même largement utilisée comme intermédiaire dans la préparation de différents produits notamment pour la synthèse finale de dérivés de thiéno[3,2-c]pyridine de formule générale :

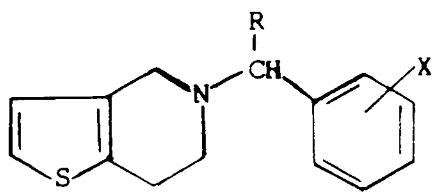

Ia

dans laquelle R représente l'hydrogène ou un radical

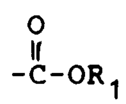

dans lequel $R_1$ représente un radical alkyle en $C_1$-$C_4$ tel que méthyle et X représente l'hydrogène ou un atome d'halogène tel que chlore ainsi que de leurs sels pharmaceutiquement acceptables.

De tels dérivés de thiéno[3,2-c]pyridine, qu'ils soient sous forme de mélange racémique ou d'énantiomères séparés, ont été décrits notamment dans les brevets français Nos. 2 215 948, 2 530 247 et 2 612 929.

Ces composés se sont révélés particulièrement intéressants pour leurs applications thérapeutiques notamment pour leurs propriétés antiagrégantes plaquettaires et anti-thrombotiques.

Parmi les dérivés de thiéno[3,2-c]pyridine de formule la les plus intéressants, on peut citer la (chloro-2 benzyl)-5 tétrahydro-4,5,6,7 thiéno[3,2-c]pyridine ou ticlopidine (DCI) ainsi que l'$\alpha$-(tétrahydro-4,5,6,7 thiéno[3,2-c]pyridyl-5) (chloro-2 phényl)acétate de méthyle plus particulièrement sous la forme de son énantiomère dextrogyre ou clopidogrel (DCI).

On a décrit dans le brevet FR-A-2 508 456, un procédé de préparation de la (thiényl-2)-2 éthylamine comportant les étapes suivantes :

a) la transformation du nitro-1 (thiényl-2)-2 éthylène en thiényl-2 acétaldoxime par hydrogénation catalytique sur palladium pour donner la thiényl-2 acétaldoxime que l'on isole de son milieu réactionnel, et

b) la réduction de cette oxime en (thiényl-2)-2 éthylamine par hydrogénation en présence d'un catalyseur, à savoir le nickel de Raney.

Si la deuxième étape peut être réalisée dans des conditions analogues à celles généralement utilisées pour la réduction d'imines, c'est-à-dire dans un solvant alcoolique saturé d'ammoniac, la mise en oeuvre de la première étape se révèle plus délicate. En effet, le nitro-1 (thiényl-2)-2 éthylène est sensible à la fois aux milieux acides et basiques.

En milieu basique, ce composé peut conduire à la formation de composés de dimérisation ou de polymérisation tandis qu'en milieu acide, on peut observer l'ouverture du noyau thiophène pour donner des mercaptans (poison de catalyseur) et la déshydratation de l'oxime formée en nitrile correspondant.

Selon une mise en oeuvre décrite dans ce brevet, on effectue notamment la réduction du nitro-1 (thiényl-2)-2 éthylène, en milieu acide acétique/éthanol 75/25, en présence de 1% de palladium sous forme métallique et à une dilution de 5%, ce qui fournit, avec un rendement de 60%, la thiényl-2 acétaldoxime sous forme huileuse.

On sait, par ailleurs, que la fonction oxime peut être généralement obtenue par réaction d'une fonction aldéhyde avec l'hydroxylamine, ce qui, dans le cas présent, laisserait supposer l'obtention de la thiényl-2 acétaldoxime à partir du thiényl-2 acétaldéhyde.

Une méthode de préparation de cet aldéhyde a été décrite dans J. Org. chem. 17 pp. 1183-1186 (1952) et 18 pp. 878-881 (1953), selon laquelle on fait réagir le thiényl-2 carboxaldéhyde avec le chloroacétate de méthyle en présence de méthylate de sodium dans l'éther éthylique, ce qui fournit le thiényl-2 glycidate de méthyle avec un rendement de 64%.

On saponifie alors ce dérivé glycidique au moyen d'hydroxyde de sodium dans l'éthanol puis on décarboxyle par chauffage en thiényl-2 acétaldéhyde, les meilleurs rendements étant obtenus après acidification préalable du milieu réactionnel.

Le rendement global de ce procédé de préparation du thiényl-2 acétaldéhyde, à savoir 12% à partir du thiényl-2 carboxaldéhyde, laisse supposer par conséquent que la thiényl-2 acétaldoxime ne pourrait être obtenue à partir du thiényl-2 acétaldéhyde ainsi préparé qu'avec un rendement de 12% maximum quelle que soit la méthode utilisée pour la mise en oeuvre de l'hydroxylamine et du thiényl-2 acétaldéhyde.

La recherche d'un procédé industriel pour la préparation de la thiényl-2 acétaldoxime, mettant en oeuvre des intermédiaires de synthèse d'accès facile et peu onéreux ainsi qu'un rendement satisfaisant en produit final reste d'un intérêt incontestable.

On a maintenant découvert que le thiényl-2 glycidate d'isopropyle permet de préparer la thiényl-2 acétaldoxime en question avec des rendements de l'ordre de 90%, c'est-à-dire de loin supérieurs à ceux qui pourraient être obtenus selon la technique antérieure.

De plus, on a trouvé que le thiényl-2 glycidate d'isopropyle peut être obtenu lui-même de manière remarquablement avantageuse et avec des rendements très importants puisque supérieurs à 95%.

En conséquence, l'invention se rapporte au thiényl-2 glycidate d'isopropyle en tant que produit industriel nouveau, utile notamment comme intermédiaire de synthèse par exemple pour la préparation de la thiényl-2 acétaldoxime.

Selon l'invention, on prépare le thiényl-2 glycidate d'isopropyle en faisant réagir, dans l'isopropanol et à température ambiante, le thiényl-2 carboxaldéhyde avec un haloacétate d'isopropyle, par exemple le chloroacétate d'isopropyle, en présence d'un isopropylate de métal alcalin préférentiellement l'isopropylate de sodium.

Selon une mise en oeuvre préférée de l'invention, on obtient le thiényl-2 glycidate d'isopropyle par introduction d'un mélange de thiényl-2 carboxaldéhyde et de chloroacétate d'isopropyle dans un mélange isopropanol/isopropylate de métal alcalin, par exemple l'isopropylate de sodium.

Comme mentionné précédemment, le thiényl-2 glycidate d'isopropyle peut donner accès à la thiényl-2 acétaldoxime.

En conséquence, un autre objet de l'invention se rapporte à la préparation de la thiényl-2 acétaldoxime par mise en oeuvre d'un procédé selon lequel :

a) on saponifie le thiényl-2 glycidate d'isopropyle au moyen d'un hydroxyde de métal alcalin, ce qui fournit le glycidate de métal alcalin correspondant, et

b) on traite ce glycidate de métal alcalin, après séparation ou non de son milieu de préparation, avec une solution aqueuse d'un sel d'hydroxylamine, chlorhydrate ou sulfate par exemple, la réaction ayant lieu à température ambiante, ce qui fournit la thiényl-2 acétaldoxime désirée.

Généralement, la saponification a lieu dans un solvant organique tel qu'un alcool $C_1$-$C_4$ et à une température comprise entre la température ambiante et la température de reflux du milieu réactionnel, préférentiellement à une température de l'ordre de 40 à 60°C.

En outre, l'hydroxyde de métal alcalin est de préférence l'hydroxyde de sodium ou de potassium et le sel d'hydroxylamine est préférentiellement sous forme de chlorhydrate.

Selon une mise en oeuvre préférée, on prépare la thiényl-2 arétaldoxime sans isolement du thiényl-2 acétaldéhyde formé transitoirement.

Selon une variante particulièrement avantageuse de l'invention, on réalise la préparation de la thiényl-2 acétaldoxime via le thiényl-2 glycidate d'isopropyle dans le milieu même où cet ester est préparé.

En conséquence, selon une variante de l'invention, on prépare la thiényl-2 acétaldoxime selon un procédé mettant en oeuvre les étapes suivantes :

a) on fait réagir, dans l'isopropanol et à température ambiante, le thiényl-2 carboxaldéhyde, pour obtenir, transitoirement et sans isolement, le thiényl-2 glycidate d'isopropyle, et

b) on saponifie l'ester obtenu, au moyen d'un hydroxyde de métal alcalin et à une température comprise entre la température ambiante et la température de reflux du milieu, puis on traite à température ambiante, avec une solution aqueuse d'un sel d'hydroxylamine, ce qui fournit, après réaction de décarboxylation, l'oxime désirée.

Comme indiqué précédemment, le thiényl-2 glycidate d'isopropyle peut être utilisé pour la préparation des dérivés de thiéno[3,2-c] pyridine de formule Ia.

En conséquence, l'invention se rapporte également au thiényl-2 glycidate d'isopropyle en tant qu'intermédiaire pour la synthèse finale des dérivés de thiéno[3,2-c]pyridine de formule Ia, en particulier pour la synthèse de la ticlopidine et du clopidogrel.

Par exemple, on peut préparer ces composés de formule Ia au départ de la thiényl-2 acétaldoxime obtenue elle-même selon l'invention à partir du thiényl-2 glycidate d'isopropyle, par mise en oeuvre d'un procédé comportant la suite d'étapes ci-après :

(a) on hydrogène cette oxime à une pression d'hydrogène comprise entre 1 et 100 atmosphères ($10^5$ et $10^7$ Pa), à une température comprise entre 20 et 100°C et en présence d'un agent basique tel que l'ammoniac et d'un catalyseur métallique tel que le nickel de Raney, le charbon palladié ou le noir de platine, pour obtenir la (thiényl-2)-2 éthylamine,
(b) on fait réagir le composé ainsi obtenu avec le formaldéhyde ou le paraformaldéhyde, généralement 1 à 1,5 mole par mole de composé de départ, la réaction s'effectuant par chauffage à une température de 70 à 90°C, pour obtenir la formimine de la (thiényl-2)-2 éthylamine,
(c) on fait réagir le composé ainsi formé, avec un acide tel que l'acide chlorhydrique et à une température comprise entre 40 et 60°C, pour obtenir un sel de tétrahydro-4,5,6,7 thiéno[3,2-c] pyridine, par exemple le chlorhydrate, que l'on traite éventuellement au moyen d'un agent basique tel qu'un hydroxyde de métal alcalin pour régénérer le dérivé de thiéno[3,2-c] pyridine sous forme basique,
(d) on fait réagir, en présence d'un accepteur d'acide, par exemple un carbonate ou bicarbonate de métal alcalin et éventuellement dans des conditions de transfert de phase, la tétrahydro-4,5,6,7 thiéno[3,2-c] pyridine sous forme de base ou de sel, avec un composé de formule générale :

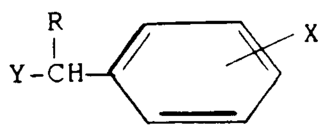

II

dans laquelle R et X ont la même signification que précédemment et Y représente un atome d'halogène tel que chlore ou brome ou un groupe alkylsulfonate en $C_1$-$C_4$ tel que méthanesulfate ou arylsulfonate en $C_6$-$C_{10}$ tel que benzènesulfonate ou p-toluènesulfonate, la réaction ayant lieu de préférence à une température de 60 à 90°C pour obtenir le composé désiré de formule Ia que l'on peut transformer en sel pharmaceutiquement acceptable par réaction avec un acide organique ou inorganique approprié.

Lorsque R est différent d'hydrogène, le composé de formule Ia est obtenu sous forme de mélange d'énantiomères. Ces énantiomères peuvent être séparés selon des méthodes connues par exemple par recristallisation de sels d'acides optiquement actifs ou par application d'une technique chromatographique.

Généralement, les étapes (a) à (d) ci-dessus peuvent être réalisées en solution aqueuse ou dans un solvant organique polaire tel qu'un alcool en $C_1$-$C_4$.

L'étape (a) s'effectue dans un solvant saturé de préférence en ammoniac, tandis que l'étape (b) peut également se dérouler dans l'eau c'est-à-dire à partir d'une solution aqueuse de formaldéhyde.

Quant à l'étape (d), celle-ci peut être réalisée dans un solvant inerte tel qu'un alcool en $C_1$-$C_4$, le N,N-diméthylformamide, un éther tel que le tétrahydrofuranne ou l'éther isopropylique, un ester tel que l'acétate d'éthyle.

En outre, par "conditions de transfert de phase", on entend des conditions réactionnelles se déroulant en système de solvants diphasique et en présence d'un catalyseur de transfert de phase.

Comme système de solvants diphasique on peut citer notamment les systèmes hydrocarbure/eau ou éther/eau. Quant au catalyseur de transfert de phase, il peut s'agir par exemple d'un sel d'ammonium quaternaire, d'un sel de phosphonium ou d'un éther couronne.

Les étapes (a) à (d) ci-dessus sont des étapes connues ayant été décrites dans les brevets FR-2 215 948, 2 508 456 et JP-63-101385 (Chem. Abstr. 109, 92980q).

Les exemples non limitatifs suivants illustrent l'invention :

EXEMPLE 1

Préparation du thiényl-2 glycidate d'isopropyle

Dans un ballon de 250 ml, on prépare une solution d'isopropylate de sodium en additionnant 3,6g de sodium à 140 ml d'isopropanol puis en chauffant progressivement jusqu'à 80°C durant 30 minutes. On laisse le milieu sous agitation jusqu'à consommation complète du sodium. On refroidit le milieu réactionnel à 20°C et on introduit un mélange de 16,8 g de thiényl-2 carboxaldéhyde et de 22,55 g de chloroacétate d'isopropyle durant 10 minutes. On agite la solution pendant 25 minutes à cette température puis on refroidit vers 5 à 10°C. On ajoute alors ce milieu réactionnel à un mélange de 50 g de glace, de 150 ml d'eau et de 2 ml d'acide acétique. On extrait avec d'abord 150 ml puis avec 2 fois 75 ml de dichlorométhane, on regroupe les phases organiques et on les sèche sur sulfate de sodium. Après filtration et évaporation des solvants, on obtient 30,85 g d'une huile orangée que l'on distille sous pression réduite pour donner 29,6 g d'un liquide incolore.

De cette manière, on obtient le thiényl-2 glycidate d'isopropyle.

Rendement : 93%.

P.E. : 105°C (0,5 mm Hg ou 66,5 Pa)

$n_D^{20}$= 1,5170

Spectre I.R. (film) : 3100-3000 (-CH aromatique); 1735 (COO); 1250 (C-O-C)$cm^{-1}$

Spectre RMN$^1$H ($CDCl_3$) (300 MHz) de l'énantiomère (E) δ = 1,29 (6H,d,$CH_3$); 3,64 (1H,d,CH-O); 4,28 (1H,d,CH-O); 5,12 [1H,m,$\underline{CH}(CH_3)_2$]; 6,97 (1H,m,CH aromatique); 7,17 (1H,d,CH aromatique); 7,27 (1H,d,CH aromatique) ppm.

Spectre RMN$^1$H ($CDCl_3$) (300 MHz) de l'énantiomère (Z) δ = 1,12 (6H,d,$CH_3$); 3,82 (1H,d,CH-O); 4,78 (1H,d,CH-O); 4,97 [1H,m,$\underline{CH}(CH_3)_2$]; 6,97 (1H,m,CH aromatique); 7,17 (1H,d,CH aromatique); 7,27 (1H,d,CH aromatique) ppm.

EXEMPLE 2

Préparation de la thiényl-2 acétaldoxime

Dans un ballon, on introduit 30,85 g de thiényl-2 glycidate d'isopropyle dans 140 ml d'isopropanol. On ajoute alors en 5 minutes 25,7 g d'une solution d'hydroxyde de sodium à 35% P/V puis on agite la solution durant 2 heures à 50°C. On refroidit vers 25°C et on introduit alors en 10 minutes, 16,15 g de chlorhydrate d'hydroxylamine dissous dans 20 ml d'eau. On maintient le milieu sous agitation pendant 30 minutes puis on l'ajoute à 150 ml d'eau et 150 ml de dichlorométhane. On extrait avec 2 fois 100 ml de dichlorométhane, on regroupe les phases organiques et on les sèche sur sulfate de sodium. On filtre et on évapore le solvant.

De cette manière, on obtient 20,16 g de thiényl-2 acétaldoxime cristallisé.

Rendement : 95%

P.F. : 92°C

Spectre I.R. (KBr) : 3400-3100 (OH); 3100-3000 (=CH aromatique); 2855 (CH=N); 1655 (C=N)$cm^{-1}$

Spectre RMN$^1$H (DMSO-$d_6$) (300 MHz) δ = 3,68 [2H,d,$CH_2$ (Z)]; 3,84 [2H,d,$CH_2$ (E)]; 6,62-6,91 [6H,m, protons aromatiques(Z) + (E)]; 7,37 [1H,t,C(H)=N]; 10,71 [H,s, OH(Z)]; 11,1 [1H,s,OH (E)] ppm.

EXEMPLE 3

Préparation de la thiényl-2 acétaldoxime

Dans un réacteur de 2,5 l on prépare une solution d'isopropylate de sodium en additionnant 36 g de sodium à 1,4 l d'isopropanol et en chauffant progressivement jusqu'à 80°C durant 30 minutes. On laisse le milieu sous agitation jusqu'à consommation complète du sodium puis on refroidit à 20°C.

On introduit alors en 30 minutes, un mélange de 168 g de thiényl-2 carboxaldéhyde et de 12,9 g de chloroacétate d'isopropyle. On agite la solution durant 25 minutes à cette température puis on ajoute, en 30 minutes, 257 g d'une solution aqueuse d'hydroxyde de sodium à 35%. On agite durant 2 heures à 50°C, on refroidit vers 25°C puis on introduit en 30 minutes, 161,5 g de chlorhydrate d'hydroxylamine dissous dans 200 ml d'eau. On maintient le milieu sous agitation pendant 30 minutes puis on l'ajoute à 1,5 l d'eau et 1,5 l de dichlorométhane.

On extrait avec deux fois 1l de dichlorométhane, on regroupe les phases organiques et on sèche sur sulfate de sodium.

Après filtration et évaporation des solvants, on obtient 191 g de thiényl-2 acétaldoxime cristallisée.

Rendement : 90%

P.F. 92°C

Spectre RMN$^1$H (DMSO-$d_6$) (300 MHz) δ = 3,68 ppm [2H,d,$CH_2$ (Z)]; 3,84 ppm [2H,d,$CH_2$ (E)]; 6,62-6,91 ppm [3H,m, protons aromatiques]; 7,37 ppm [1H,t,C(H)=N]; 10,71 ppm [H,s,OH(Z)]; 11,1 ppm [1H,s,OH (E)].

Les exemples suivants illustrent la préparation de la ticlopidine et du clopidogrel.

EXEMPLE A

Préparation du chlorhydrate de (chloro-2 benzyl)-5 tétrahydro-4,5,6,7 thiéno [3,2,c]pyridine

a) (Thiényl-2)-2 éthylamine

On purge à l'azote un autoclave de 300 ml avant mise sous vide et l'on introduit 100 ml d'une solution d'isopropanol saturé d'ammoniac (11,1 g), 7,5 g de thiényl-2 acétaldoxime préparée selon l'exemple 3 ci-dessus et 0,7 g de catalyseur au nickel de Raney (1 g humide).

On place l'ensemble sous une pression de 20 bars d'hydrogène (2 x $10^6$ Pa) et l'on élève la température jusqu'à 50°C en 20 minutes tout en maintenant une agitation du milieu réactionnel. On effectue périodiquement l'appoint d'hydrogène à 20 bars au fur et à mesure de sa consommation jusqu'à refus d'absorption (environ 6 heures).

On filtre alors le catalyseur, on rince avec de l'isopropanol et on effectue une analyse du produit de départ par chromatographie en phase gazeuse avec étalonnage interne.

De cette manière, on obtient la (thiényl-2) -2 éthylamine.

Rendement : 91,5%.

b) Formimine de la (thiényl-2)-2 éthylamine

Dans un ballon tricol de 500 ml muni d'un thermomètre, d'un réfrigérant, d'un agitateur mécanique et d'une ampoule d'introduction, on charge 181 g de (thiényl-2)-2 éthylamine. On additionne, en 10 minutes, 130 g d'une solution aqueuse de formol à 35%. Dès le début de l'addition du formol, le milieu réactionnel prend un aspect laiteux et sa température croit régulièrement pour atteindre 90°C. On agite durant 30 minutes avant d'introduire 300 ml de toluène et 100 ml d'une solution aqueuse de chlorure de sodium à 10%. Après décantation, on extrait à nouveau la phase aqueuse avec 2 fois 100 ml de toluène, on rassemble les phases toluéniques et on les lave avec 2 fois 100 ml d'eau saturée en chlorure de sodium. Après évaporation du toluène, on obtient 198 g d'huile qui cristallise à 0°C pour donner la formimine de la (thiényl-2)-2 éthylamine avec un rendement de 100%.

P.F. = 49°C.

Spectre RMN[1]H (DMSO,$d_6$) (300 MHz)

$\delta$ = 2,7 - 3,0 (4H,m,$CH_2$-$CH_2$); 3,46 (2H,s, -N-$CH_2$-N); 6,8-7,2 (3H,m,protons aromatiques) ppm.

c) Chlorhydrate de tétrahydro-4,5,6,7 thiéno [3,2-c] pyridine

Dans un ballon muni d'un agitateur mécanique, d'un réfrigérant et d'un thermomètre, on charge 433 ml d'une solution 6,4 N d'acide chlorhydrique dans le N,N-diméthylformamide. On chauffe la solution à 40°C et on additionne, en 30 minutes, 198,7g de formimine de la (thiényl-2)-2 éthylamine tout en maintenant la température du milieu réactionnel entre 45 et 60°C.

On maintient sous agitation pendant 30 minutes à 60°C puis on refroidit à 0°C. On filtre le précipité, on lave avec 300 ml de toluène puis on sèche à 60°C sous vide, ce qui donne un solide blanc.

De cette manière, on obtient 227 g de chlorhydrate de tétrahydro-4,5,6,7 thiéno [3,2-c] pyridine avec un rendement de 93%.

P.F. : 226°C.

Spectre RMN[1]H (DMSO,$d_6$) (300 MHz)

$\delta$ = 3,04 - 3,35 (4H,m); 4,12 (2H,s); 6,92 (1H,d); 7,46 (1H,d); 9,40 (2H,s) ppm.

On obtient la tétrahydro-4,5,6,7 thiéno [3,2-c] pyridine par basification du chlorhydrate correspondant au moyen de 60 ml d'hydroxyde de sodium aqueux puis extraction avec 3 fois 70 ml de dichlorométhane. On regroupe les phases organiques, on les lave avec 80 ml d'eau puis on concentre ce qui fournit la tétrahydro-4,5,6,7 thiéno [3,2-c] pyridine.

P.E. : 70-75°C (0,5 mm Hg ou 66,5 Pa)

Rendement : 100%

Spectre RMN ($CDCl_3$) (300 MHz) $\delta$ = 2,10 (1H,s); 2,7-3,2 (4H,m); 3,9 (2H,s); 6,72 (1H,d); 7,06 (1H,d) ppm.

d) Chlorhydrate de (chloro-2 benzyl)-5 tétrahydro-4,5,6,7 thiéno [3,2-c] pyridine

Dans un ballon tricol de 250 ml muni d'un thermomètre, d'un réfrigérant et d'un agitateur mécanique, on charge 26,1 g de tétrahydro-4,5,6,7 thiéno [3,2-c]pyridine, 80 ml d'éthanol et 19 g de bicarbonate de sodium. On ajoute alors 30,2 g de chlorure d'O-chlorobenzyle et l'on chauffe vers 75-80°C pendant 1 heure. On évapore le milieu réactionnel et on reprend le résidu avec 200 ml d'éther isopropylique. On ajoute alors, à 100 ml d'éthanol, la

(chloro-2 benzyl)-5 tétrahydro-4,(,6,7 thiéno [3,2-c] pyridine obtenue. Par addition de 20 ml d'acide chlorhydrique concentré à la solution éthanolique, on obtient après chauffage à reflux et refroidissement du milieu, 46,6 g de chlorhydrate de (chloro-2 benzyl)-5 tétrahydro-4,5,6,7 thiéno [3,2-c] pyridine qui cristallise.
P.F. : 210°C.
Rendement : 83%.
Spectre RMN$^1$H ($CDCl_3$) (300 MHz)$\delta$ = 3,1-4,9 (8H,m); 6,6 (2H,d); 7,8 (4H,m) ppm.

EXEMPLE B

Préparation de l'hydrogénosulfate de l'α-(tétrahydro-4,5,6,7 thiéno [3,2-c] pyridyl-5) (chloro-2 phényl)-acétate de méthyle dextrogyre

a) Chloro-1 (chloro-2 phényl)-acétate de méthyle

On mélange 93,3 g (0,5 mole) d'acide chloro-2 mandélique pur avec 208 g (1 mole) de pentachlorure de phosphore et on chauffe progressivement. La réaction démarre à 60°C et la température monte à 100°C.

On chauffe encore durant 2 h entre 120 et 130°C. Lorsque le dégagement de gaz chlorhydrique a cessé, on ramène à température ambiante, distille à la trompe à eau puis reprend par 200 ml de méthanol.

On chauffe durant 2 h à reflux. On concentre le milieu réactionnel au moyen d'un évaporateur rotatif et on reprend au chlorure de méthylène et à l'eau. On décante, sèche sur sulfate de magnésium anhydre et chasse le chlorure de méthylène au moyen d'un évaporateur rotatif ce qui fournir 121 g d'ester brut que l'on distille entre 80 et 90°C sous une pression de 0,15 mm Hg (19,95 Pa).

De cette manière, on obtient 54,8 g de chloro-1 (chloro-2 phényl) acétate de méthyle, dosé à 90% en CLHP (chromatographie liquide haute pression).
Rendement : 45%.

b) α-(Tétrahydro-4,5,6,7 thiéno [3,2-c] pyridyl-5) (chloro-2 phényl)-acétate de méthyle racémique

On introduit dans 80 ml de solvant, 6 g de carbonate de potassium, 7 g de tétrahydro-4,5,6,7 thiéno [3,2-c] pyridine distillée (ou 15 g de carbonate de potassium et 8,8 g de chlorhydrate de tétrahydro-4,5,6,7 thiéno [3,2-c] pyridine recristallisée) puis 12 g de chloro-1 (chloro-2 phényl)-acétate de méthyle.

On maintient le mélange sous agitation à la température indiquée pendant le temps indiqué. On élimine le solvant sous pression réduite et on verse sur le résidu 50 à 100 ml d'eau et 100 à 130 ml d'acétate d'éthyle. On sépare la phase organique et on retraite la phase aqueuse au moyen de 30 à 50 ml d'acétate d'éthyle.

On réunit les phases organiques, on les lave à l'eau et on refroidit dans un bain à - 10°C. On introduit alors dans le milieu, un mélange de 20 g de glace et 10 ml d'acide chlorhydrique concentré. Le produit final précipite. Après une heure on filtre et on sèche.

De cette manière, on obtient l'α-(tétrahydro-4,5,6,7 thiéno[3,2-c]pyridyl-5) (chloro-2 phényl)-acétate de méthyle racémique. P.F. : 130-140°C (acétate d'éthyle/isopropanol).

Selon les solvants, température de temps de réaction ci-dessous, on a obtenu les rendements suivants :

| Solvant | Température (°C) | Temps de réaction (h) | Rendement (%) |
|---|---|---|---|
| Tétrahydrofuranne | 65 | 20 | 80 |
| Acétate d'éthyle | 77 | 20 | 81 |
| Ether isopropylique | 65 | 20 | 79 |
| N,N-diméthylformamide | 75 | 3 | 69 |

c) Sel de l'acide l-camphosulfonique-10 de l'α-(tétrahydro-4,5,6,7 thiéno [3,2-c] pyridyl-5) (chloro-2 phényl)-acétate de méthyle

On dissout 32 g (0,0994 mole) d'α-(tétrahydro-4,5,6,7 thiéno [3,2-c] pyridyl-5) (chloro-2 phényl)-acétate de méthyle racémique dans 150 ml d'acétone. On ajoute 9,95 g (0,0397 mole) d'acide camphosulfonique-10 monohydraté lévogyre. On abandonne le milieu homogène à température ambiante. Après 48 heures, il y a apparition de quelques cristaux. On concentre le milieu réactionnel à 50 ml et abandonne 24 heures à température ambiante. On filtre les cristaux obtenus, on lave à l'acétone et on sèche (rendement : 55% par rapport au racémique de départ).

On redissout, dans un minimum d'acétone bouillante (50 ml), les cristaux ainsi obtenus puis, après refroidissement, on filtre, lave à l'acétone et sèche.

De cette manière, on obtient le sel de l'acide l-camphosulfonique-10 de l'α-(tétrahydro-4,5, 6,7 thiéno [3,2-c] pyridyl-5) (chloro-2 phényl)-acétate de méthyle.
Rendement : 88%.
P.F. : 165°C.
$\alpha_D^{20}$ = + 24,75° (c = 1,68g/100 ml; méthanol)

d) α-(Tétrahydro-4,5,6,7 thiéno [3,2-c] pyridyl-5) (chloro-2 phényl)-acétate de méthyle dextrogyre

On ajoute 800 ml d'une solution aqueuse de bicarbonate de sodium à une suspension de 200 g de sel de l'acide l-camphosulfonique-10 de l'α-(tétrahydro-4,5,6,7 thiéno [3,2-c] pyridyl-5) (chloro-2 phényl)-acétate de méthyle dans 800 ml de dichlorométhane. Après agitation, on décante la phase organique, sèche sur sulfate de sodium et élimine le solvant sous pression réduite pour obtenir l'α-(tétrahydro-4,5,6,7 thiéno [3,2-c] pyridyl-5) (chloro-2 phényl)-acétate de méthyle dans 800 ml de dichlorométhane. Après agitation, on décante la phase organique, sèche sur sulfate de sodium et élimine le solvant sous pression réduite pour obtenir l'α-(tétrahydro-4,5,6,7 thiéno [3,2-c] pyridyl-5) (chloro-2 phényl)-acétate de méthyle dextrogyre sous forme d'une huile incolore.

e) Hydrogénosulfate de l'α-(tétrahydro-4,5,6,7 thiéno [3,2-c] pyridyl-5) (chloro-2 phényl)-acétate de méthyle dextrogyre

On dissout dans 500 ml d'acétone refroidie dans la glace, le résidu obtenu précédemment et on ajoute, goutte à goutte, 20,7 ml d'acide sulfurique concentré (93,64%; d = 1,83).

On isole, par filtration, le précipité apparu, on lave avec 1000 ml d'acétone puis on sèche en étuve à vide à 50°C.

De cette manière, on obtient 139 g d'hydrogénosulfate de l'α-(tétrahydro-4,5,6,7 thiéno [3,2-c] pyridyl-5) (chloro-2 phényl)-acétate de méthyle dextrogyre sous forme de cristaux blancs.
P.F. : 184°C.
$\alpha_D^{20}$ = + 55,1° (c = 1,891 g/100 ml; méthanol).

## Revendications

1. Thiényl-2 glycidate d'isopropyle de formule :

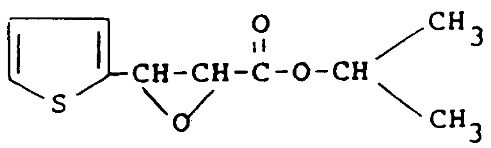

I

2. Procédé de préparation du thiényl-2 glycidate d'isopropyle, caractérisé en ce que l'on fait réagir dans l'isopropanol et à température ambiante, le thiényl-2 carboxaldéhyde avec un haloacétate d'isopropyle, en présence d'un isopropylate de métal alcalin, ce qui fournit le composé désiré.

3. Procédé selon la revendication 2, caractérisé en ce que l'haloacétate d'isopropyle est le chloroacétate d'isopropyle et l'isopropylate de métal alcalin est l'isopropylate de sodium.

4. Procédé de préparation de la thiényl-2 acétaldoxime, caractérisé en ce que :

   a) on saponifie dans un solvant organique le thiényl-2 glycidate d'isopropyle au moyen, d'un hydroxyde de métal alcalin et à une température comprise entre 40 et 60°C, pour obtenir le glycidate de métal alcalin correspondant,
   b) on traite ce glycidate de métal alcalin après séparation ou non de son milieu de préparation, avec une solution aqueuse d'un sel d'hydroxylamine, la réaction ayant lieu à température ambiante ce qui fournit, après réaction de décarboxylation, le composé désiré.

5. Procédé de préparation de la thiényl-2 acétaldoxime, caractérisé en ce que :

   a) on fait réagir, dans l'isopropanol et à température ambiante, : le thiényl-2 carboxaldéhyde avec un haloacétate d'isopropyle en présence d'un isopropylate de métal alcalin pour obtenir transitoirement et sans isolement, le thiényl-2 glycidate d'isopropyle,

b) on saponifie dans un solvant organique le glycidate obtenu, au moyen d'un hydroxyde de métal alcalin à une température comprise entre 40 et 60°C, puis on traite, à température ambiante, avec une solution aqueuse d'un sel d'hydroxylamine, ce qui fournit, après réaction de décarboxylation, le composé désiré.

**6.** Procédé de préparation de dérivés de thiéno[3,2-c] pyridine de formule générale :

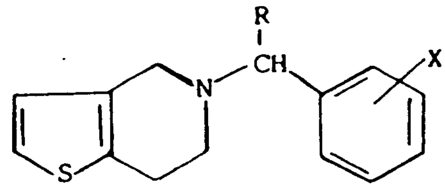

Ia

ainsi que de leurs sels pharmaceutiquement acceptables, dans laquelle
R représente l'hydrogène ou un radical

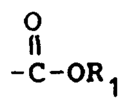

dans lequel $R_1$ représente un radical alkyle en $C_1$-$C_4$ et X représente l'hydrogène ou un atome d'halogène, caractérisé en ce que :

a) on fait réagir, dans l'isopropanol et à température ambiante, le thiényl-2 carboxaldéhyde avec un haloacétate d'isopropyle en présence d'un isopropylate de métal alcalin pour obtenir transitoirement et ses isolement, le thiényl-2 glycidate d'isopropyle,
b) on saponifie dans un solvant organique le glycidate obtenu, au moyen d'un hydroxyde de métal alcalin et à une température comprise entre 40 et 60°C, puis on traite, à température ambiante, avec une solution aqueuse d'un sel d'hydroxylamine, ce qui fournit, après réaction de décarboxylation, la thiényl-2 acétaldoxime,
(c) on hydrogène cette oxime à une pression d'hydrogène comprise entre $10^5$ et $10^7$ Pa, à une température comprise entre 20 et 100°C et en présence d'un agent basique et d'un catalyseur métallique pour obtenir la (thiényl-2)-2 éthylamine,
(d) on fait réagir le dérivé d'éthylamine obtenu avec le formaldéhyde ou le paraformaldéhyde, la réaction s'effectuant par chauffage à une température de 70 à 90°C pour obtenir la formimine de la (thiényl-2)-2 éthylamine,
(e) on fait réagir le composé ainsi formé, avec un acide et à une température comprise entre 40 et 60°C, pour obtenir un sel de tétrahydro-4,5,6,7 thiéno[3,2-c] pyridine, que l'on traite éventuellement au moyen d'un agent basique pour régénérer le dérivé de thiéno[3,2-c] pyridine sous forme basique,
(f) on fait réagir, en présence d'un accepteur d'acide et éventuellement dans des conditions de transfert de phase, la tétrahydro-4,5,6,7 thiéno[3,2-c] pyridine sous forme de base ou de sel avec un composé de formule générale :

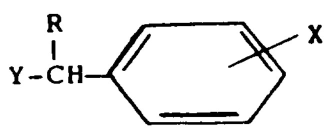

II

dans laquelle R et X ont la même signification que précédemment et Y représente un atome d'halogène tel que chlore ou brome ou un groupe alkylsulfonate en $C_1$-$C_4$ ou arylsulfonate en $C_6$-$C_{10}$, la réaction ayant lieu à une température de 60 à 90°C pour obtenir le dérivé désiré de thiéno[3,2-c] pyridine sous forme basique, que l'on peut, lorsque R est différent d'hydrogène, séparér en ses énantiomères sous forme basique, le dérivé basique ainsi obtenu étant si nécessaire transformé en sel pharmaceutiquement acceptable par réaction avec un acide organique ou inorganique approprié.

7. Procédé selon l'une des revendications 4 à 6, caractérisé en ce que l'hydroxyde de métal alcalin est l'hydroxyde de sodium ou de potassium et le sel d'hydroxylamine est le chlorhydrate ou le sulfate.

8. Procédé selon la revendication 6, caractérisé en ce que le substituant X du composé de formule II représente le chlore et le substituant R du composé de formule II représente l'hydrogène ou un radical méthoxycarbonyle.

9. Procédé selon la revendication 6, pour la préparation de la (chloro-2 benzyl)-5 tétrahydro-4,5,6,7 thiéno [3,2-c]pyridine.

10. Procédé selon la revendication 6, pour la préparation de l'énantiomère dextrogyre de l'α-(tétrahydro-4,5,6,7 thiéno[3,2-c] pyridyl-5) (chloro-2 phényl)-acétate de méthyle.

**Claims**

1. Isopropyl-2-thienylglycidate of the formula

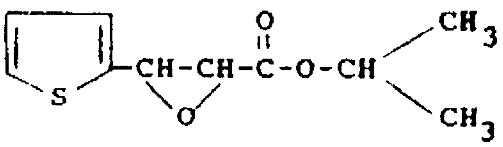

I

2. Process for preparing isopropyl-2-thienylglycidate, characterised in that 2-thienylcarboxaldehyde is reacted in isopropanol and at ambient temperature with an isopropylhaloacetate, in the presence of an alkali metal isopropoxide, thereby obtaining the desired compound.

3. Process according to claim 2, characterised in that the isopropylhaloacetate is isopropylchloroacetate and the alkali metal isopropoxide is sodium isopropoxide.

4. Process for preparing 2-thienylacetaldoxime, characterised in that:

a) isopropyl-2-thienylglycidate is saponified in an organic solvent using an alkali metal hydroxide at a temperature of between 40 and 60°C, in order to obtain the corresponding alkali metal glycidate,
b) this alkali metal glycidate, which may or may not be separated from its preparation medium, is treated with an aqueous solution of a hydroxylamine salt, the reaction taking place at ambient temperature, so as to produce the desired compound after a reaction of decarboxylation.

5. Process for preparing 2-thienylacetaldoxime, characterised in that:

a) 2-thienylcarboxaldehyde is reacted, in isopropanol and at ambient temperature, with an isopropylhaloacetate in the presence of an alkali metal isopropoxide in order to obtain isopropyl-2-thienylglycidate transiently, without isolation thereof,
b) the glycidate obtained is saponified in an organic solvent using an alkali metal hydroxide at a temperature between 40 and 60°C, then treated at ambient temperature with an aqueous solution of a hydroxylamine salt, in order to obtain the desired compound after a reaction of decarboxylation.

**6.** Process for preparing thieno[3,2-c]pyridine derivatives of the general formula:

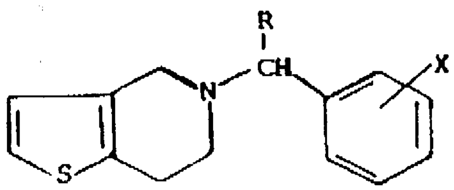

Ia

and the pharmaceutically acceptable salts thereof, wherein
R represents hydrogen or a

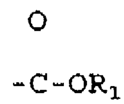

wherein $R_1$ denotes a $C_{1-4}$-alkyl radical and X denotes hydrogen or a halogen atom, characterised in that:

(a) 2-thienylcarboxaldehyde is reacted, in isopropanol and at ambient temperature, with an isopropylhaloacetate, in the presence of an alkali metal propoxide, so as to obtain the isopropyl-2-thienylglycidate transiently and without isolation thereof,
(b) the glycidate obtained is saponified in an organic solvent, using an alkali metal hydroxide, at a temperature between 40 and 60°C then treated at ambient temperature with an aqueous solution of a hydroxylamine salt, thereby obtaining 2-thienylacetaldoxime, after a reaction of decarboxylation,
(c) this oxime is hydrogenated at a hydrogen pressure of between $10^5$ and $10^7$ Pa, at a temperature of between 20 and 100°C, in the presence of a basic agent and a metal catalyst in order to obtain 2-(2-thienyl)-ethylamine,
(d) the ethylamine derivative obtained is reacted with formaldehyde or paraformaldehyde, the reaction being carried out by heating to a temperature of 70 to 90°C in order to obtain 2-(2-thienyl)-ethylamine formimine,
(e) the compound thus formed is reacted with an acid at a temperature of between 40 and 60°C in order to obtain a 4,5,6,7-tetrahydrothieno[3,2-c]pyridine salt, which is optionally treated with a basic agent in order to regenerate the thieno[3,2-c]pyridine derivative in basic form,
(f) the 4,5,6,7-tetrahydrothieno[3,2-c]pyridine is reacted in the form of a base or salt, in the presence of an acid acceptor and optionally under phase transfer conditions, with a compound of general formula:

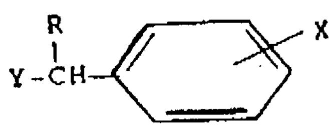

II

wherein R and X are as hereinbefore defined and Y denotes a halogen atom such as chlorine or bromine or a $C_{1-4}$-alkylsulphonate or $C_{6-10}$-arylsulphonate group, the reaction taking place at a temperature of 60 to 90°C in order to obtain the desired thieno[3,2-c]pyridine derivative in basic form, which, if R is other than hydrogen, may be separated into its enantiomers in basic form, the basic derivative thus obtained being, if necessary, converted into a pharmaceutically acceptable salt by reaction with a suitable organic or inorganic acid.

**7.** Process according to one of claims 4 to 6, characterised in that the alkali metal hydroxide is sodium or potassium hydroxide and the hydroxylamine salt is the hydrochloride or sulphate.

**8.** Process according to claim 6, characterised in that the substituent X of the compound of formula II represents chlorine and the R substituent of the compound of formula II denotes hydrogen or a methoxycarbonyl radical.

**9.** Process according to claim 6 for the preparation of 5-(2-chlorobenzyl)-4,5,6,7-tetrahydrothieno[3,2-c]pyridine.

**10.** Process according to claim 6 for the preparation of the dextrorotatory enantiomer of methyl-α-(4,5,6,7-tetrahydro-5-thieno[3,2-c]pyridyl) (2-chlorophenyl)acetate.

## Patentansprüche

**1.** 2-Thienyl-glycidsäureisopropylester der Formel:

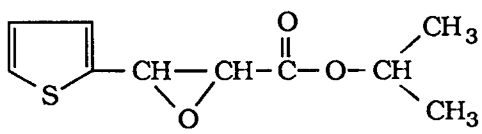

I

**2.** Verfahren zur Herstellung von 2-Thienyl-glycidsäureisopropylester, **dadurch gekennzeichnet**, daß man 2-Thienylcarboxaldehyd in Isopropanol und bei Raumtemperatur und in Gegenwart von einem Alkalimetallisopropylat mit einem Halogenessigsäureisopropylester zur Bildung der gewünschten Verbindung umsetzt.

**3.** Verfahren nach Anspruch 2, **dadurch gekennzeichnet**, daß der Halogenessigsäureisopropylester Chloressigsäureisopropylester ist und als Alkalimetallisopropylat Natriumisopropylat eingesetzt wird.

**4.** Verfahren zur Herstellung von 2-Thienyl-acetaldoxim, **dadurch gekennzeichnet**, daß man:

a) 2-Thienyl-glycidsäureisopropylester mit einem Alkalimetallhydroxid in einem organischen Lösungsmittel und bei einer Temperatur zwischen 40 und 60°C verseift zur Bildung des entsprechenden Alkalimetallglycidats,
b) das Alkalimetallglycidat gegebenenfalls nach seiner Abtrennung aus dem Herstellungsmedium mit einer wäßrigen Lösung eines Hydroxylaminsalzes umsetzt, wobei die Reaktion bei Raumtemperatur erfolgt, was nach der Decarboxylierungsreaktion zu der gewünschten Verbindung führt.

**5.** Verfahren zur Herstellung von 2-Thienyl-acetaldoxim, **dadurch gekennzeichnet**, daß man:

a) 2-Thienyl-carboxaldehyd in Isopropanol und bei Raumtemperatur und in Gegenwart eines Alkalimetallisopropylats mit einem Halogenessigsäureisopropylester umsetzt, so daß man 2-Thienyl-glycidsäureisopropylester als Zwischenprodukt erhält, das nicht isoliert wird,
b) den erhaltenen Glycidsäureester in einem organischen Lösungsmittel mit Hilfe eines Alkalimetallhydroxids bei einer Temperatur zwischen 40 und 60°C verseift und dann bei Raumtemperatur mit einer wäßrigen Lösung eines Hydroxylaminsalzes behandelt, so daß man nach der Decarboxylierungsreaktion die gewünschte Verbindung erhält.

**6.** Verfahren zur Herstellung der Thieno[3,2-c]pyridin-Derivate der allgemeinen Formel:

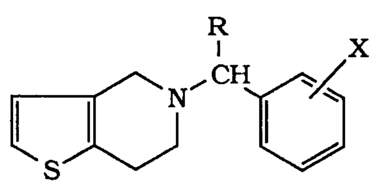

Ia

sowie von deren pharmazeutisch annehmbaren Salzen, worin
R Wasserstoff oder eine Gruppe der Formel

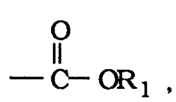

in der $R_1$ eine $C_1$-$C_4$-Alkylgruppe darstellt, und X Wasserstoff oder ein Halogenatom bedeuten, **dadurch gekennzeichnet**, daß man:

a) 2-Thienyl-carboxaldehyd in Isopropanol und bei Raumtemperatur in Gegenwart eines Alkalimetallisopropylats mit einem Halogenessigsäureisopropylester umsetzt, so daß man den 2-Thienyl-glycidsäureisopropylester als Zwischenprodukt erhält, der nicht isoliert wird,
b) man den erhaltenen Glycidsäureester in einem organischen Lösungsmittel mit Hilfe eines Alkalimetallhydroxids und bei einer Temperatur zwischen 40 und 60°C verseift und dann bei Raumtemperatur mit einer wäßrigen Lösung eines Hydroxylaminsalzes behandelt, so daß man nach der Decarboxylierungsreaktion 2-Thienyl-acetaldoxim erhält,
c) man dieses Oxim bei einem Wasserstoffdruck zwischen $10^5$ und $10^7$ Pa bei einer Temperatur zwischen 20 und 100°C in Gegenwart eines basischen Mittels und eines Metallkatalysators hydriert zur Bildung von 2-(2-Thienyl)-ethylamin,
d) man das erhaltene Ethylaminderivat mit Formaldehyd oder Paraformaldehyd durch Erhitzen auf eine Temperatur von 70 bis 90°C umsetzt zur Bildung von 2-(2-Thienyl)-ethylamin-formimin,
e) man die in dieser Weise gebildete Verbindung mit einer Säure und bei einer Temperatur zwischen 40 und 60°C umsetzt, so daß man ein Salz von 4,5,6,7-Tetrahydro-thieno[3,2-c]pyridin erhält, welches man gegebenenfalls mit einem basischen Mittel behandelt zur Bildung des Thieno[3,2-c]pyridin-Derivats in Form der Base,
f) man 4,5,6,7-Tetrahydro-thieno[3,2-c]pyridin in Form einer Base oder des Salzes in Gegenwart eines Säureakzeptors und gegebenenfalls unter Phasentransferbedingungen mit einer Verbindung der allgemeinen Formel:

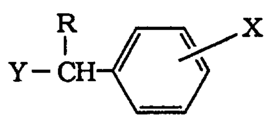

II

in der R und X die oben angegebenen Bedeutungen besitzen und Y ein Halogenatom, wie ein Chlor- oder Bromatom, oder eine $C_1$-$C_4$-Alkylsulfonatgruppe oder eine $C_6$-$C_{10}$-Arylsulfonatgruppe bedeutet, bei einer Temperatur von 60 bis 90°C umsetzt, so daß man das gewünschte Thieno[3,2-c]pyridin-Derivat in Form der Base erhält, welches man, wenn R von Wasserstoffverschieden ist, in seine optischen Enantiomeren in Form der Base auftrennen kann, welches in dieser Weise erhaltene basische Derivat erforderlichenfalls durch Reaktion mit einer geeigneten organischen oder anorganischen Säure in ein pharmazeutisch annehmbares Salz umgewandelt wird.

7. Verfahren nach einem der Ansprüche 4 bis 6, **dadurch gekennzeichnet**, daß das Alkalimetallhydroxid Natrium- oder Kaliumhydroxid und das Hydroxylaminsalz das Hydrochlorid oder das Sulfat ist.

8. Verfahren nach Anspruch 6, **dadurch gekennzeichnet**, daß der Substituent X der Verbindung der Formel II Chlor und der Substituent R der Verbindung der Formel II Wasserstoff oder eine Methoxycarbonylgruppe bedeuten.

9. Verfahren nach Anspruch 6 zur Herstellung von 5-(2-Chlor-benzyl)-4,5,6,7-tetrahydro-thieno[3,2-c]pyridin.

10. Verfahren nach Anspruch 6 zur Herstellung des rechtsdrehenden Enantiomeren des $\alpha$-(4,5,6,7-Tetrahydrothieno[3,2-c]pyrid-5-yl)-(2-chlor-phenyl)-essigsäuremethylesters.